# EUROPEAN PATENT APPLICATION

(11) **EP 4 497 381 A1**
(43) Date of publication of application: **29.01.2025**
(21) Application number: 23188236.6
(22) Date of filing: 27.07.2023
(51) Int. Cl.: A61B 5/16, A61B 5/00

(54) **SYSTEM AND METHOD FOR ASSESSING NEUROCOGNITIVE FUNCTIONING**

(71) Applicant: Max-Planck-Gesellschaft zur Förderung der Wissenschaften e.V., 80539 München (DE)
(72) Inventor: SPOORMAKER, Victor, 80805 Munich (DE); FIETZ, Julia, 80469 Munich (DE)
(74) Representative: LKGLOBAL Lorenz & Kopf Patentanwalt Attorney at Law PartG mbB

(57) **Abstract**

The present invention relates to a system (100) for assessing neurocognitive functioning, comprising a task component (101) configured to provide a task, in particular a cognitive task to a user; a capture component (102) configured to capture biomarker data of a biomarker of the user; and a processing component (103) configured to generate a biomarker response profile based on the biomarker data. The present invention further relates to a method for assessing neurocognitive functioning, comprising the steps of providing a task to a user; capturing biomarker data of a biomarker of the user; generating a biomarker response profile based on the biomarker data.

## Description

The invention relates to a system and method for assessing neurocognitive functioning, specifically capturing a biomarker of a user in response to a cognitive task.

Neurocognitive functioning is the ability to perform various cognitive tasks, such as memory, attention, language, and executive functions. Neurocognitive functioning can be affected by various factors, such as aging, brain injury, disease, or medication. Biomarkers are measurable indicators of biological processes or conditions that can be used to diagnose, monitor, or predict a disease pattern. Pupillary response is one example of a biomarker that can reflect neurocognitive functioning.

Pupil response can be defined as the change in the size of the pupil in response to different stimuli, such as light, affective stimuli , attentional stimuli or stimuli manipulating cognitive load, and can be measured using pupillometry, which comprises recording and analysing the pupil diameter over time. Pupillometry can provide information about the activity of the brain regions and pathways involved in pupil control, such as the retina, the optic nerve, the midbrain and brainstem, the autonomic nervous system, i.e., subcortical and cortical pathways (e.g. dACC, orbitofrontal cortex etc.), and the cortex.

Different methods for assessing neurocognitive functioning using pupil response as a biomarker exist. For example, pupil light reflex (PLR) can be used which captures the constriction of the pupil in response to light stimulation. PLR can be used to assess the function of the sensory and motor pupillary pathways, as well as the intrinsically photosensitive retinal ganglion cells that mediate non-image-forming responses to light. PLR can also be modulated by factors such as alertness, circadian rhythm, and sleepiness.

PLR may be influenced by external factors, such as ambient light, stimulus intensity, duration, and wavelength. This may require a high level of standardized and controlled conditions to ensure reliable and valid measurements. PLR can also be affected by physiological factors, such as age, sex, iris colour, or medication. Therefore, PLR may require more individualized and normalized data to account for inter- and intra-subject variability. PLR can have a reduced sensitivity to cognitive stimuli. Therefore, PLR may not capture the subtle or complex changes in neurocognitive functioning that are associated with different types of tasks, processes, or states.

Consequently, informativeness and predictiveness of PLR as an indicator of diagnosis, prognosis, or treatment response for various neurological or psychiatric conditions can be limited.

WO2017190033A1 discloses a method for detecting brain trauma. A light flash is used to take a series of images of the pupil of the patient and the position and relative size of the pupil in each image is determined. From the series of images and the relative size of the pupil or a rate of change in pupil size, a degree of risk of brain trauma can be determined. Furthermore, ambient light can be included in the measurement to influence the effect of the light stimulus.

Alternatively to PLR, the spontaneous fluctuation of the pupil diameter in the absence of external stimuli can be captured (pupillary unrest). Pupillary unrest can be used to measure the activity of the sympathetic and parasympathetic branches of the autonomic nervous system, which regulate arousal and stress levels.

Alternatively, pupil dilation, i.e., enlargement of the pupil in response to cognitive or emotional stimuli, can be captured. Pupil dilation can be used to measure the level of cognitive load, attention, or interest in a task or situation. Pupil dilation can also reflect emotional arousal, valence, or empathy.

Pupil dilation is the enlargement of the pupil in response to cognitive or emotional stimuli. Cognitive load is the amount of mental effort required to perform a task or process information. Pupil dilation can be used as a measure of cognitive load, because it reflects the activity of the brain regions and pathways involved in cognitive control, such as the locus coeruleus-norepinephrine system (LC-NE) 1. The LC-NE system is responsible for regulating arousal, attention, and stress levels, and it modulates the activity of other brain regions involved in cognitive functions, such as memory, learning, and decision making.

Pupil dilation increases with increasing cognitive load, such as when performing more difficult or complex tasks, memorizing more information, or solving problems. Thereto, reference can be made to Fietz J, et. al., Pupillometry tracks cognitive load and salience network activity in a working memory functional magnetic resonance imaging task (Hum Brain Mapp 43: 665-680, 2022), Robison MK, Unsworth N: Pupillometry tracks fluctuations in working memory performance (Atten Percept Psychophys 81: 407-419, 2019) and Unsworth N, Robison MK, Tracking working memory maintenance with pupillometry (Atten Percept Psychophys 80: 461-484, 2018). Pupil dilation can also vary depending on the type of cognitive task, such as verbal, spatial, or numerical. Pupil dilation can also be influenced by factors such as individual differences, motivation, emotion, fatigue, or noise .

It is therefore an object of embodiments of the present invention to overcome or at least reduce some or all of the above described disadvantages of the known neurocognitive assessment systems by providing a system for assessing neurocognitive function which can provide an increased accuracy and efficiency based on a correlation of cognitive load and a specific biomarker response.

According to a first aspect the invention relates to a system for assessing neurocognitive functioning, comprising a task component configured to provide a task, in particular a cognitive task, to a user and a capture component configured to capture biomarker data of a biomarker of the user and a processing component configured to generate a biomarker response profile based on the biomarker data.

The capture component may comprise an eye-tracking device is configured to measure the user's pupil dilation during or after the completion of the task. The processing component may be configured to receive data relating to the pupil dilation from the eye-tracking device and to analyze the data using one or more algorithms or models that are based on the relationship between pupil dilation and LC activity. The processing component may be configured to output feedback to the user or a clinician based on the level of cognitive impairment. In one embodiment, the capture device, in particular comprising the eye-tracking device may be integrated in a smartphone. The user may download an application that provides an interface to the task component or the task component itself. The task component can provide various cognitive tasks, such as memory recall, arithmetic, spatial reasoning, and/or attention switching. The user can perform the tasks on the smartphone screen, in particular while holding the smartphone is disposed at a fixed distance from the user's eyes. The task component can be configured to present a task, in particular present the task visually and/or auditorily to the user. The smartphone may comprise the capture component in form of a camera which can be configured to capture images of the user's eyes and measure their pupil dilation using image processing techniques. The smartphone may comprise the processing component in form of a smartphone processor that can be configured to analyze the biomarker data, specifically pupil dilation data.

The task component can comprise a user interface, wherein the user interface is configured capture a user input and provide the user input to the processing component. The user input can relate to a task feedback, i.e., correspond to a response requested by the task. The user interface can be configured to provide the task to the user in a perceivable manner, i.e., visually, auditorily and/or haptically. The user interface may be configured to provide feedback to the user based on a task response. Thereto, the user interface can be configured to acknowledge a user input by providing a confirmation signal, i.e., sound, visual indicator and/or haptic feedback signal (vibration). The user interface may further be configured to structure a task, respectively task conditions by providing a feedback signal. Thus, a beginning and/or end of separate blocks, task conditions and/or tasks can be indicated to the user. The user interface can be configured visually, auditorily or haptically capture a user input. For example, the user can voice a feedback, look at a specific element, press a button and/or provide a touch response. The user interface can comprise a graphical interface component providing a platform interface to the user, wherein the platform interface can provide control functions to the user. Control functions can relate to at least one of the following functions: initiate a task, initiate a set of tasks, initiate biomarker capture, initiate task performance analysis, initiate storage of task results and/or analysis results etc.

The processing component can be configured to utilize machine learning algorithms or neural networks that are trained on data from healthy and/or impaired individuals. The smartphone application can provide feedback to the user or a clinician based on the level of cognitive impairment, such as a score, a graph, a message, or an alert.In another embodiment, the capture component, preferably comprising a display device and the eye-tracking device can be integrated in a VR headset. The user can wear the VR headset and experience various immersive environments that provide cognitive tasks, such as navigating a maze, solving puzzles, or interacting with virtual characters. Additionally, all tasks providable via the task component, respectively a standard visual or sound output can be provided via the VR headset. The VR headset can measure the user's pupil dilation using specific capture components, preferably infrared sensors and/or cameras that can be embedded in the headset. The VR headset can communicate with the processing component which analyze the pupil dilation data using statistical methods or artificial intelligence techniques that are based on the physiological principles of pupillometry. The VR headset can provide feedback to the user or a clinician based on the level of cognitive impairment, such as an audiovisual cue, a notification, or a report. The processing component can be provided by a cloud service.

The assessment of neurocognitive functioning can in particular identify a deviation from a baseline level of neurocognitive functioning. The baseline level can be established by evaluating a plurality of non-pathological individuals, i.e., a suitable control group. Neurocognitive malfunctioning, i.e., deviation from a predetermined norm can be associated with psychopathology across multiple disorders including anxiety and mood disorders, obsessive compulsive disorder, schizophrenia, anorexia, respectively bulimia nervosa, substance use disorders or personality disorders.

Furthermore, neurocognitive malfunctioning can be linked to a plurality of psychopathological syndromes, such as rumination, dissociation or schizotypy in addition to perceived subjective burden and illness chronicity.

Furthermore, baseline neurocognitive functioning can be predictive of a treatment response in pharmaco- and psychotherapy.

The biomarker, in particular the pupil size, can be captured task-evoked constituting physiological readout for neurocognitive functioning. The system according to the present invention can provide pupil response profiles during working memory processing of a user. Herein, profiles of a heterogenous sample including healthy and unmedicated users can be used to establish a baseline for identifying pathological biomarker response profiles.

A relationship between a neurocognitive pathology, specifically Alzheimer's Disease (AD) and pupil dilation can vary based on the disease stage. The system can be used to detect and/or diagnose a plurality of neurocognitive disorders and/or diseases. The system can be configured to determine a cognitive impairment of a user which can be used as an indicator across psychiatric and neurological diseases. Specifically, the system can be configured to determine a decline in cognitive functioning (cognitive impairment). Thereto specific pupillometric protocols can be used. The present system can provide AD screening or diagnosis based on the evaluation of the captured biomarker response profile, specifically the pupil size. The task component can be configured to periodically provide a task to the user and/or provide a reminder to the user to complete a task, respectively a task session, comprising a plurality of tasks. Herein, the system can be configured to monitor a progression of the neurocognitive state of the user. This can achieve the advantage that impact of a medication can be determined, and/or that a threshold level to initiate medication can be monitored. This can achieve the advantage of increasing the effectiveness of a medication and thereby prognosis of the user as the medication may have an increased effectiveness in earlier stages of a psychiatric and/or neurological disease. Examples of these medications can include Lecanemab (BAN2401) and/or Donanemab, i.e., medication to reduce harmful protein deposits in the brain (amyloid beta, amyloid plaques).

The modules can be integrated to form a single system component. Alternatively, the system can be modularized and/or decentralized. Specifically, the capture component can be in range to capture the biomarker from the user. The task component can be at a first remote location and can be configured to provide task data to a local interface configured to interact with the user to provide the task. Additionally and/or alternatively, the processing component can be at a second remote location. This can achieve the advantage that components relating to capturing the biomarker and presenting the cognitive task can be general purpose items, e.g., a smartphone, a computer or a VR-headset and the specialized task component and processing component can be located elsewhere.

The capture component and/or the processing component can form a high-resolution pupillometry system that can capture small changes in pupil diameter and account for factors such as eye movements, blinks, or head position. This achieves the advantage of capturing pupil response accurately and reliably. The capture component can comprise video-based eye trackers, infrared cameras, and/or wearable devices.

The processing component can be configured to determine a profile parameter based on the biomarker response profile and to assess the neurocognitive capability of the user based on the profile parameter. This can achieve the advantage that the amount of data necessary for evaluation can be reduced. Specifically, the profile parameter can be a condensed representation of the biomarker response profile, i.e., specifying a key characteristic of the profile. The profile parameter can be assessed on a scale. The processing component can be configured to determine a z-score based on the biomarker data, respectively the biomarker response profile. Preferably, the pupillometry data can be z-scored, comprising standardizing with a mean value or the standard deviation. Thereby, observations from different distributions that may have different units or scales can be compared. The processing component can be configured to identify outlier values that are unusually high or low compared to the rest of the data. Herein, the z-score may be used to errors, and/or anomalies in the biomarker data, respectively biomarker response profile. The processing component can be configured to determine probabilities and percentiles using the standard normal distribution. For example, the processing component can be configured to determine a probability of a user having pathologic neurological impairment, respectively neurological disease. A Z-score can represent an efficient measure to analyse the biomarker response profile, since only the mean and/or standard deviation of the biomarker response profile may need to be calculated. Z-scores can be a measure that enables efficient comparison between different datasets as they have a mean of zero and a standard deviation of one. Also Z-scores can be compared within a single biomarker response profile by looking at their relative positions.

Preferably the scale can comprise threshold values relating to the potential of the profile parameter indicating a pathology. The profile parameter can be provided separate from the cognitive task, in particular to a review component configured to provide diagnostic data based on the biomarker data, respectively biomarker response profile, preferably in relation to the cognitive tasks or a plurality of cognitive tasks performed by the user. Herein, the plurality of cognitive tasks can be immediately consecutive, i.e., without a significant time delay. For example, the tests can be continuous, in particular such that the type of tests remains the same but the cognitive difficulty is increased. Additionally or alternatively, the tasks can be completed at different times, e.g., with time intervals of minutes, hours or days.

The profile parameter may correspond to a statistical measure and/or a parameter of a mathematical function, in particular a function fitted to the biomarker response profile. The processing component can be configured to automatically determine the statistical measure by processing the biomarker response profile. The statistical measure can be based on a mathematical model, wherein parameters of the mathematical model can correlate to specific properties of the biomarker, biomarker data, respectively biomarker response profile. For example, fitted parameters according to the statistical measure can correlate to a pupil parameter: pupil size, pupil size variance, latency of pupillary constriction to light, constriction amplitude (decrease or increase), redilation after light offset, maximum velocity of constriction (MCV) and/or maximum constriction acceleration (MCA).

Furthermore, the processing component can be configured to determine a mean dilation, i.e., a mean increase rate of the pupil, an acceleration of the dilation, a median dilation, a mean acceleration of the dilation, and/or a median acceleration of the dilation. Additionally or alternatively, the mean, median and/or variance of pupil size across a time interval, preferably a 1-minute block, can be determined. The processing component can be configured to associate a biomarker response profile as a response to target stimuli.

The processing component may be configured to determine a difference measure and to assess the neurocognitive capability of the user based on the difference measure. The difference measure can be a Euclidean distance or a set of Euclidean distance of the biomarker response profile to a reference profile. The reference profile can be a statistical average of a plurality of biomarker response profiles. The reference profile can be associated with a healthy control group. Alternatively, the reference profile can be associated with a control group having a specific pathology. Furthermore, the difference measure can comprise a plurality of difference values, wherein each difference value is associated with a specific reference profile. Accordingly, not only the distance to a healthy control profile, but also the distance to a pathologic control profile, preferably specific pathologies, can be determined. A similarity, i.e., reduced distance to one of the control profiles, i.e., a similarity of the profiles, can indicate am an affiliation of the captured biomarker response profile to that specific reference profile. The processing component can be configured to assign the captured biomarker response profile to a specific reference profile, and thereby to a specific medical condition based on the difference measure.

The difference measure can correspond to at least one of the following:
difference of the profile parameter to a predetermined baseline value;
difference of a fitted curve parameter to a predetermined fitted curve parameter;
similarity of the biomarker response profile to a reference profile;
scaling of the biomarker response profile in comparison to a reference profile;
scaling of a parameter fitted to the biomarker response profile in comparison to a reference parameter.

The processing component can be configured to determine a neurocognitive capability measure based on the biomarker response profile and/or the difference measure. The neurocognitive capability measure can be an indicator of neurocognitive capability of the user. This can be a generalized measure based on biomarker response profile and/or the performance in the task, i.e., time to completion, errors, speed per individual component of the task, corrections etc. The processing component can be configured to provide the neurocognitive capability measure to the user and/or to a medical data interface. This enables remote diagnosis and/or remote monitoring of the user's cognitive function, in particular as a marker for neurological pathologies. The processing component can be configured to assess the neurocognitive capability measure in relation to previously determined neurocognitive capability measures to determine an onset, a progression and/or improvement of a neurocognitive pathology.

The processing component can be configured to compare the difference measure and/or the biomarker response profile against a set of predetermined classification criteria (values). The processing component may further be configured to associate the biomarker response profile with a neurocognitive capability class, which in particular corresponds to a pathological cognitive impairment of the user. The processing component can be configured to base associating the biomarker response profile with a neurocognitive capability class on the outcome of the comparison with the predetermined classification criteria. Thereby, the biomarker response profile can be linked to a specific category increasing efficiency in generating a diagnosis for the user. The classification criteria can comprise a comparison of neurocognitive scores with normative values of these scores. The normative values can represent a non-pathological, i.e., healthy condition of the user and/or a pathological, i.e., non-healthy condition of the user. Based on the neurocognitive scores falling within a predetermined, preferably continuous, interval encompassing the respective normative value, the biomarker response profile can be classified by the condition represented by the respective normative value. In other words, the neurocognitive score can fall within a value band associated with a healthy condition or within a further value band associated with a pathological condition indicating a neurocognitive impairment.

The processing component can be configured to map the profile parameter to a value on a cognitive capability scale to provide a cognitive capability measure. The cognitive capability measure can indicate an instantaneous cognitive capability, an averaged cognitive capability or a peak cognitive capability. The cognitive capability measure can be separate from a classification of the biomarker response profile to a specific pathological class. For example, the cognitive capability measure can be determined independent of a link to a specific normative value, value band and its assignment to a specific neurocognitive capability class. The scale can be individualized with respect to further bio indicators of the user. These bio indicators can be used as a weight to the profile parameter such that a weighted profile parameter can be mapped to a generalized cognitive capability scale. The mapping of the profile parameter can comprise applying a transfer function transforming the biomarker response profile and/or profile parameter to a cognitive capability measure that is removed from measurement specifics of the actual biomarker. As such the cognitive capability measure can be compared to other cognitive capability measures which may rely on different biomarkers or may be obtained by alternative systems.

The processing component can be configured to update the cognitive capability measure while the cognitive task is provided, preferably while the cognitive task is performed by the user. This achieves the advantage that processing of the biomarker can already be executed while the task is still performed by the user. This can provide real time updates of the cognitive capability measure and/or reduce processing delays once the task is complete. For example, the processing component may generate the biomarker response profile, profile parameter or cognitive capability measure based on a partially complete dataset and update the respective quantity as more data is captured.

The processing component can be configured to update the biomarker response profile periodically or continuously. A continuous update can provide insights on fluctuations of the biomarker during the task. Periodical updates can increase computation efficiency and provide an increase in stability, i.e., reduction in fluctuation of values, of the output. Herein, an average can be applied to increase confidence and readability of the output, i.e., the biomarker response profile, profile parameter and/or cognitive capability measure.

The processing component can be configured to update the biomarker response profile while the cognitive task is provided, preferably while the cognitive task is performed by the user.

The processing component can be configured to update the neurocognitive capability measure periodically or continuously, in particular when a task is completed, sub-part of the task is completed or at least part of the task is completed. This can achieve the advantage that the neurocognitive capability measure can indicate performance for part of the task. In particular a variance with respect to the type of test or cognitive load of the test can be determined. The neurocognitive capability measure may not be provided to the user during the task so as to not influence performance of the user for subsequent task.

The processing component may be configured to provide the neurocognitive capability measure. Preferably, the neurocognitive capability measure can be provided in relation to reference values, in particular comprising a baseline value and/or threshold values indicating a predetermined likelihood of the current neurocognitive capability measure being a non-baseline value. This can increase efficiency and reduce the specialization required to assess the results. In particular, analysis of the biomarker response profile can be provided by the processing component to remove the need for manual mapping of the biomarker response profile or profile parameter to a cognitive capability measure. The processing component can be configured to assess the neurocognitive capability measure on a continuous scale. The scale can comprise reference values associated with specific conditions. Examples of specific conditions can comprise a healthy condition and/or pathological conditions, in particular different stages of a pathological condition representing a neurocognitive impairment. Each condition may be associated with a mean value and/or a value band.

The processing component can be configured to provide a confidence measure corresponding to a variability of the neurocognitive capability measure, in particular a variance and/or error measure. This can provide an estimate on the precision of the biomarker response profile, respectively profile parameter and thereto also pertaining to the cognitive capability measure. An automatic classification of the biomarker response profile to a neurocognitive capability class can be conditional on a confidence measure threshold value, i.e., the confidence may need to be high enough and/or the variance may not exceed a predetermined variance threshold value. The confidence measure may be provided in conjunction with the biomarker response profile, profile parameter and/or cognitive capability measure.

The system may comprise a review component which may be configured to provide a representation of the biomarker response profile, in particular a numerical and/or graphical representation. The review component may display the biomarker response profile continuously and update a graphical representation during the performance of the task. Visualizing also the confidence measure can provide information relating to the precision of the obtained biomarker response profile already during the task. The review component may also be configured to provide a representation of the difference measure. Information provided via the review component may not be visible to the user during the task.

The review component may be configured to provide a representation of the neurocognitive capability measure and/or the confidence measure. The review component can therefore achieve the advantage of an efficient information display based on the captured data.

The review component can be configured to provide the representation of the neurocognitive capability measure periodically or continuously, in particular parallel to the task component providing the cognitive task. Thereby allowing an assessment during providing the cognitive task. Thereby, flexible continuation of the task can be achieved. Additionally or alternatively, adjustment of the task, in particular the cognitive load can be performed. The processing component can continue the task according to an error measure, i.e., variance, value threshold. For example, when the biomarker response profile during the task shows a high variance the task may be continued until a lower variance is achieved. This can increase accuracy of the cognitive capability measure.

The review component may be configured to provide task data to the task component. Preferably, the task component can be configured to modify the current cognitive task based on the task data. The task data can comprise a modified task and/or timing data relating to, for example, the display time of a current task, repetition of a current task and/or modified response time relating to the task. Response time can be the time interval given to the user to respond to a sub task until the next sub task is provided.

The task component may be configured to adjust a cognitive task difficulty of the cognitive task and/or provide a subsequent task with a cognitive task difficulty different to the cognitive task difficulty of the previous task. The cognitive task difficulty can be adjusted quantitatively such that an expected relation between cognitive load and biomarker response profile, profile parameter and/or cognitive capability measure can be determined. Specifically, the processing component can correlate the cognitive difficulty to an expectation range for the biomarker response profile and/or profile parameter. For example, an increase in cognitive load by a predetermined margin can determine an expected biomarker response profile, profile parameter and/or cognitive capability measure. The biomarker response profile can be determined over a plurality of tasks, wherein each task corresponds to a predetermined cognitive load value. A biomarker response profile captured for a specific task may form part of a combined biomarker response profile which comprises multiple biomarker response profiles concatenated according to increasing cognitive load.

The system can comprise a database module configured to store a plurality of biomarker response profiles and/or cognitive capability measures. The database module can serve as a resource to the processing component to determine the difference measure based on the stored plurality of biomarker response profiles and/or the stored plurality of cognitive capability measures.

The processing component may be configured to compare a present biomarker response profile to the plurality of response profiles and to generate a relative measure of the biomarker response profile indicating a relation of the present biomarker response profile to the plurality of response profiles. Herein, the plurality of biomarker response profiles may form a similarity band, in particular without relying on determining a specific reference profile. In other words, the plurality of biomarker response profiles can be considered as a band of values. The band may have a varying spread in relation to the cognitive load or generally the quantity it is captured over. Herein, the processing component can be configured to assign a quality associated with the plurality of biomarker response profiles to the present biomarker response profile based on an overlap of the captured biomarker response profile with the value set spanned by the plurality of biomarker response profiles. This can reduce the processing complexity as a statistical measure may not need to be determined. The present biomarker response profile can be assessed based on similarity to profiles in the database. The processing component may provide common biomarkers of the profiles forming a similarity band to the present biomarker response profile, profile parameter and/or cognitive capability measure. Thus, based on similarities cognitive functioning of the user corresponding to the present biomarker response profile can be assessed. A similarity diagnostic can be provided via an interactive interface which may provide a plurality of subsets of the plurality of stored biomarker response profiles according to specific comparison parameters.

The processing component can be configured to compare a present cognitive capability measure to the plurality of cognitive capability measures and to generate a measure, in particular a relative measure, of the present cognitive capability measure indicating a relation of the present cognitive capability measure to the plurality of cognitive capability measures. Thus, manual assessment of similarity can be replaced by an automatic evaluation decreasing the effort to evaluate the biomarker response profile. Herein, the database can comprise biomarker response profile associated with a specific cognitive capability measure such that the processing component can compare cognitive capability measures instead of biomarker response profiles. This can increase computing efficiency since the cognitive capability measures can comprise less complex data than the underlying biomarker response profiles. Furthermore, the cognitive capability measure can be a means for providing diagnostic information, in particular with reduced complexity. Thereby, the cognitive capability measure can be perceived as an indicator for cognitive impairment by the user directly, preferably via a corresponding smartphone app, and/or in particular with limited or without consultation of a specialist.

Additionally or Alternatively, the processing component can compare biomarker response profiles, in particular on a single datapoint level which may increase computational load and/or increase precision of the comparison, i.e., a closer match to an existing biomarker response profile may be obtained. From a match or similarity to an existing biomarker response profile respectively cognitive capability measure the processing component can infer further properties of the existing data to the presently recorded data.

The processing component can be configured to receive medical data relating to the user to which the task is provided and to provide a relevance indicator corresponding to the cognitive capability measure in relation to the medical data. The medical data can correspond to a medical condition, in particular a cognitive impairment and/or mental pathology. Additionally or alternatively, the medical data can comprise further parameter data,. The parameter data can relate to a quantitatively measurable biomarker. Specific examples are neurodegeneration markers in the cerebrospinal fluid, e.g., phospho tau, overall tau (hTau), beta-amyloid etc. Herein, the processing component may be configured to adjust a comparison dataset from the database module based on the medical data. In particular, a comparison set corresponding to similar medical data elements can be chosen, and can in particular be used to determine the difference measure. The processing module can determine a significance of the determined cognitive capability measure in relation to an existing diagnosis or a new diagnosis, preferably where the cognitive capability measure indicates a quantitative measure of the severity of a medical condition.

The task component can be configured to provide a first task and a second task and wherein the second task is configured to induce a higher cognitive load than the first task. Preferably, a set of consecutive tasks can be provided were the cognitive load increases from task to task. Alternatively, a profile of varying cognitive load can be provided. The task component may provide a calibration task between tasks of increasing cognitive load, wherein the calibration task may be configured to produce a baseline response of the biomarker, respectively a baseline biomarker response profile. Each task can correspond to a task condition.

The task component can be configured to provide a task comprising a plurality of task conditions. For example, the plurality of task conditions can comprise a plurality of N-back conditions, preferably a 0-back condition, a 1-back condition, a 2-back-condition, and/or a 3-back-condition. Conditions of the task can be provided to the user in a predetermined order. Preferably, a cognitive load of subsequently provided conditions can increase.

The N-Back task achieves the advantage of the system being able to flexibly manipulate a cognitive load. Additionally, the N-Back task can be experimentally well controllable by means of temporal fine-tuning. For example the task duration and/or task condition duration can be flexibly adapted. Also, the type of the stimuli (e-g-, letters, spatial location, etc.) and/or the length of the blocks can be controlled. The N-Back task may not require a verbal response. A response or missed response may require continuous attention and/or may generate a continuous working memory load.

The task component can be configured to provide a plurality of tasks wherein each of the tasks of the plurality of tasks has an assigned cognitive load value and wherein the task component is configured to provide the tasks based on a predetermined cognitive load profile. The cognitive load profile may quantify the cognitive load of each task and provide a specific order of higher cognitive load tasks and lower cognitive load tasks. Preferably, tasks can be provided with increasing cognitive load for subsequent tasks.

The task component can be configured to select a cognitive load profile from a plurality of cognitive load profiles. The cognitive load profile can be selected based on a profile input. Preferably, the user providing the profile input is a different user than the user performing the task such that the person performing the task is not aware of the assigned cognitive load.

The task component can be configured to generate a task based on a cognitive task template. This achieves the advantage that individualized tasks can be provided, wherein each task may be unique or at least new to the user performing the task, i.e., the user may have processed the same type of task but not the specific task generated by the task component. A cognitive task template can define the type of task, response intervals, cognitive load, means of providing the task, i.e., visually and/or audible.

The task component can be configured to select a cognitive task template from a plurality of task templates. Selecting a task template can be based on the cognitive load profile. For example, the cognitive load for a task to be selected can be defined, but the type of task can be arbitrary. Herein, the task component may provide a task according to stored biomarker response profiles and/or cognitive capability measure to increase comparability of the biomarker response profile, respectively cognitive capability measure which is to be captured corresponding to the generated task, respectively the selected cognitive task template.

The task component can be configured to select a task template and/or a task based on biomarker data and/or a biomarker response profile captured in response to a previously performed task.

The task component can be configured to select a task template and/or a task based on a predetermined expected quantitative difference in cognitive load of the respective task template and/or task, in particular in reference to a previously and/or subsequently provided task.

The task component can be configured to provide a working memory task, in particular an N-back task. Based on the established interconnection between the noradrenergic activity of the LC and the pupil and the importance of the LC-NE system for regulating the attentional status, the pupil linked individual differences in working memory can reflect regulation and dysregulation of the LC-NE system. A working memory task may provide a robust link between pupil size and cognitive load. Pupil size may increase with cognitive load across conditions in parallel with activity in the frontoparietal network which is the typically observed working memory large-scale brain network. Individual differences in working memory can predict performance in a variety of cognitive domains, such as learning and/or fluid reasoning.

The task component can be configured to provide a set of tasks and/or a set of task conditions, preferably a set of consecutive N-back conditions, more preferably a 0-back, 1-back, 2-back, and/or a 3-back condition. The task component can provide a fixation condition as part of a task or as part of a set of tasks. The task component can provide a set of conditions as a single task. The processing component can be configured to identify at least two separate biomarker response profile clusters, i.e., pupil size values of the different cognitive load conditions, from a N-back task. Specifically, the two clusters can be characterized by a reactive pattern, i.e. pupil size increasing in parallel to increasing cognitive load and a non-reactive pattern without such an increase in pupil size, in particular a step-wise increase. Each cluster can be associated with neurocognitive functioning, such as reaction times in 0-back, 2-back, cognitive flexibility, and/or inhibitory control, as well as the performance score in sustained attention.

The biomarker can correspond to the pupil size of the user, wherein the capture component may comprise a camera module configured to capture an image of the eye, preferably the iris, of the user and to determine the pupil size based on the captured image. The capture component and/or the processing component can be configured to track an eye-movement, in particular to generate a steady observation of the pupil diameter. The capture component can comprise a camera array, in particular at least two camera modules, configured to capture a stereoscopic image of the pupil. The camera module can be configured to remove distortions based on the projection of the three-dimensional eye onto the two-dimensional sensor plane, respectively sensor planes. The capture component may comprise a camera module for each pupil.

The capture component can comprise an illuminator configured to illuminate part of the user. Preferably the illuminator can be configured to illuminate the face and/or the eye of the user, more preferably configured to illuminate the iris and/or pupil of the user. This achieves the advantage that the pupil size can be captured with increased precision as the capture device, i.e., the camera module, may operate at a lower gain, e.g., lower ISO value, with an increased aperture value, i.e., increasing the depth of field, and/or with a decreased capture interval, i.e., shutter speed, reducing motion blur.

The illuminator can be configured to illuminate both eyes, irises and/or pupils of the user.

The illuminator can be configured to emit light in the visible spectrum and/or the infrared spectrum, preferably within the range of 700 nm to 1000 nm, preferably 850 nm to 950 nm. Using an infrared illumination can provide increased capture precision without triggering a light induced adaption of the pupil size. The camera module may comprise an infrared image sensor configured to capture an infrared image of the eye, iris, respectively pupil.

The capture component can interface with a smartphone app allowing users to screen for Alzheimer's disease by capturing the pupil. Specifically, the camera module can use the near-infrared spectrum. Thereby, the system can provide a scalable, smartphone based assessment tool that can be used for large-scale screenings. The system can provide the basis to establish pupil response tests as minimally-invasive and inexpensive tests to aid in the detection and understanding of diseases, e.g., Alzheimer's disease.

Using an infrared-based capture device achieves the advantage that the pupil can be easily differentiated from the iris, even in eyes with darker iris colors. Thereby, the capture component can achieve sub-millimeter accuracy across various eye colors when determining the pupil size. The capture component may be configured to simultaneously capture an infrared representation and a visible light representation of the eye, iris and/or pupil. The infrared and visual-light captures can be geometrically offset to one another. This can achieve the advantage that the stereoscopic distance between the capture device, i.e., the smartphone, and the user can be determined. The system can be configured to determine the distance between the capture component, i.e., a sensor plane of the camera and the distance to the pupil and determine the pupil size based on the distance and the pupil representation in the infrared image and/or the visual image.

By using smartphone cameras, users can easily check their own cognitive health, specifically independent of their location and/or without relying on expensive or specialized medical devices. This can increase the chance of detecting neurological conditions, in particular at early stages.

The task component can implement the smartphone to visually and/or auditorily provide instructions to the user. Herein, a high ease of use based on, for example simple instructions, large fonts, clear feedback, and voice guidance can be achieved. Furthermore the intrinsic capability of a smart device to share information can be used to easily provide feedback and/or results to medical personnel.

The capture component can be configured to continuously determine the biomarker with a sampling rate greater or equal to a predetermined threshold rate of 1 Hz, 10 Hz, 25

Hz, 50 Hz or 100 Hz. Preferably, the capture rate is at least an order of magnitude higher than the average rate of change of the biomarker in response to the cognitive load. The sampling rate can be adjusted based on a selected biomarker, i.e., according to a maximum speed of the biomarker changing its value, in particular a value change above the minimum resolution of the capture component. Consequently the capture component can be configured to capture the biomarker at a sampling rate that corresponds to the maximum rate of change of the biomarker such that two captures of the biomarker are within two resolution steps of the capture component. For example, the capture rate of a camera is high enough such that a spatial change of the biomarker, i.e. the size of the pupil between two captures does not exceed two pixels. For example, the capture rate and/or the resolution of the capture component, e.g. an image sensor, is chosen in such a fashion that capturing the movement and/or dilation of the pupil can be performed at the maximally achievable spatial resolution. A boundary between the iris and pupil may progress only by 1 pixel between two captured frames. Alternatively, the resolution can be set to be coarser, such that a shift of the boundary between iris and pupil of 2 pixels or more can be registered. Alternatively, the resolution of the capture component can be specified by a spatial resolution in millimetres. For example, the capture component can be configured to capture the pupil dilation with an accuracy of 0.5 mm, preferably 0.2 mm, more preferably 0.1 mm in reference to a pupil diameter.

The capture component can be configured to determine a relative value change of the biomarker.

The capture component can comprise a sensor, in particular an image sensor, configured to capture the biomarker data. The processing component can be configured to determine the biomarker with a relative accuracy of 5%, preferably 1%, more preferably 0.1% based on the biomarker data. The processing component can be configured to determine a correlation measure representing a consistency of the captured biomarker data, respectively biomarker response profile. Preferably the system can be configured to determine a Pearson Correlation. Herein, the system can be configured to achieve a Pearson Correlation Coefficient in the range of 0.7 to 0.9, preferably in the range above 0.84. A high accuracy in determining the biomarker can translate to a high accuracy in determining the cognitive capability measure or at least translate to a higher confidence of the biomarker response profile such that a distinction between an inconspicuous profile or a pathological profile can be made with increased certainty, reducing the risk of false positives and/or false negatives. The camera module can comprise the sensor.

The capture component can be configured to capture the biomarker during a time interval associated with the cognitive task. Thereby, the biomarker data can be directly related to the corresponding cognitive task.

The capture module can be configured to determine the biomarker for a first time interval prior to the task being provided to the user, for a second time interval during which the task is provided to the user and/or for a third time interval after the cognitive task is no longer provided to the user. This can achieve the advantage that a hysteresis of the biomarker can be captured.. Hysteresis in the biomarker response profile may occur when the biomarker not only on the current task, but also on a past task. Thereto, the biomarker may show a memory effect based on its previous states and may not respond immediately or linearly to changes in the input, i.e. a change in cognitive load. In particular, the system can determine whether the biomarker returns to a predetermined baseline level between tasks and/or whether the tasks have a compounding component, i.e., the biomarker stays at the end value captured at the tasks completion or does adjust to an intermediate value between a baseline level and a further level captured during the task. Additionally, and/or alternatively, the biomarker value of a subsequent task can be impacted by a previous task. Any biomarker capture of these intervals can form part of the biomarker response profile. A sequence of time intervals can comprise a first interval, a plurality of subsequent second intervals, where each second interval is associated with a corresponding task and/or a third time interval. Alternatively, a time interval after the task can also form a prior time interval for a subsequent task.

The sensor can be configured to track a pupil size of the user. Additionally or alternatively, the processing component and/or the capture component can be configured to track the pupil size based on the sensor input, i.e., the sensor captures a spatial range comprising all possible positions of the pupil and the processing component, respectively the capture component can be configured to track the pupil based on the sensor data.

The sensor can be configured to track an eye movement of the user. Herein the sensor and/or the capture component can account for eye movement and adjust accordingly. This can achieve the advantage that the data provided by the capture component, respectively the biomarker data, can comprise a steady representation of the biomarker increasing the efficiency of processing the biomarker data.

The processing component can be configured to determine a focus area based on the eye movement, preferably wherein the focus area is a subsection of a display area through which the task is provided.

The system can comprise a display component configured to display the task to the user. The display component can also be configured to display data provided by the review component, in particular a representation of the biomarker response profile, the profile parameter, the difference measure and/or the cognitive capability measure. The display component can be scaled according to the distance to the user such that a predetermined minimum area of the field of vision of the user is covered by the display component. The size of the display component can be scaled according to the task. This can achieve the advantage that the task can be displayed at an optimal scaling such that the visual representation of the task may not distract from the task itself.

The system can comprise a headset component configured to mount onto the head of a user and to position the display component at least partially in the field of vision of the user, preferably to dispose a primary area defined by the task within the field of vision of the user. The headset component can be a virtual reality headset or an augmented reality headset. Alternatively, the display component can be a smartphone and the headset component can be configured to hold the smartphone in the field of vision of the user. This can achieve the advantage that the display component can take up a majority of the field of vision of the user thereby reducing the influence of distractions and increasing focus on the task. Thereby, a variance of the correlation of the biomarker and the task and the corresponding cognitive load can be decreased. The combination of a smartphone and the headset component can achieve a constant scaling of the displayed task such that comparability of a plurality of biomarker response profiles captured for a plurality of users can be increased.

The headset component can be configured to at least partially block ambient light from entering the field of vision of the user. This can increase the overall focus of the user onto the task. Ambient distractions may not be visible to the user. Additionally or alternatively, the headset component may be configured to block ambient sounds to minimize distractions. The processing component may be configured to determine an influence of ambient parameters, i.e., ambient light, ambient sound and/or any ambient parameters (pressure, temperature, humidity), on the user and adjust the task accordingly. For example, an increase in variance of the biomarker may indicate an influence of ambient parameters. The task can be halted, repeated or aborted based on the variance of the biomarker exceeding a threshold value. Additionally or alternatively, ambient threshold values can be defined, wherein the processing component is may provide task data to the task component to adjust the task accordingly. The headset component may be configured to shield the user from any visual and audible input besides the task.

The display component can comprise two display units configured to display the task in conjunction with one another, where each of the display units is associated with one eye of the user. The system can be configured to process biomarker data relating to a single eye or configured to process biomarker data relating to both eyes. The number of eyes to be tracked can be determined by the type of neurological disease to be investigated.

The system can comprise a calibration component configured to determine a cognitive load of a provided cognitive task. The calibration component can thus determine a cognitive load scaling of tasks relative to one another. Additionally or alternatively the calibration component may determine an absolute cognitive load scaling of tasks relative to one another. Herein, a first baseline level and/or initial value of the biomarker response profile can be determined. Thereto a cognitive load of the task can be determined by averaging over a plurality of individuals of a control group. The calibration can be adjusted when further users fitting the control group perform the task. This may increase the overall precision of the cognitive load value assigned to a specific task. The calibration can be performed as an initialization step of the system. The calibration component may also be configured to determine reference values with respect to the present user based on datasets stored in the database module. Thereby, the calibration component may individualize a reference profile, in particular according to parameters of the user which preferably associate the user with a group of users that can be characterized by similar biomarker response profiles. As such an initial calibration can increase the precision of a classification based on the biomarker response profile and/or increase the precision of the cognitive capability measure.

The calibration component can be configured to determine a baseline level of the biomarker associated with a baseline cognitive load level.

The calibration component can be configured to determine a maximum level of the biomarker associated with a maximum cognitive load level. The calibration component can thereby determine a maximum range and/or an absolute range for the biomarker. The calibration component can be configured to weigh a biomarker response profile according to a determined baseline level and/or maximum level of the biomarker. This can increase comparability of the biomarker response profile to previously captured biomarker response profiles.

The calibration component can be configured to determine at least one intermediate level of the biomarker associated with a corresponding intermediate cognitive load level. Thereby an individualized scaling of the biomarker in reference to the cognitive load can be determined. For example, the biomarker may scale linearly or non-linearly with the cognitive load imposed by the task. The calibration component may increase the number of reference values to increase the precision in determining the scaling of the biomarker in reference to the cognitive load.

The calibration component can be configured to generate, preferably interpolate and/or extrapolate, a scale of the cognitive load, in particular wherein the cognitive load corresponds a representative biomarker value. The scale can be a quantitative scale such that the cognitive load can be increased and/or decreased by a specific value. Alternatively, the scale can be ordinal such that the cognitive load increases or decreases and the scaling may be non-linear. The processing component can be configured to determine a regression between the biomarker, e.g., the pupil size, and the cognitive load, in particular when the cognitive load is considered ordinal.

The task component can be configured to provide a set of tasks, wherein a cognitive load, preferably a cognitive load value, of subsequent tasks differs based on the quantitative scale of the cognitive load. The difference may be quantified by a predetermined value. Alternatively, an increase or a decrease of cognitive load for the subsequent task can be defined based on the cognitive load scale.

The task component can provide a task comprising task conditions that differ in a pseudo-random fashion. For example, the task conditions can be provided in an interspersed manner, e.g., 0-back, 2-back, fixation, 1-back, 2-back, 0-back, 1-back, fixation, etc. The task component can be configured to pseudo-randomize tasks provided to the user. This can increase task engagement by the user.

According to a further aspect the invention relates to a method for assessing cognitive functioning, in particular for assessing cognitive impairment in neuro-degenerative diseases using biomarker capturing, i.e. pupillometry, preferably via smartphones and/or VR/AR headsets.

The method may comprises presenting a user with one or more cognitive tasks, in particular on a display device, such as a smartphone or a virtual reality (VR) headset, and measuring the user's biomarker, e.g., the pupil dilation, during or after the completion of the tasks, in particular by means of the capture device. The capture device can comprise an eye-tracking device, such as a camera or a sensor. The method may further comprise analyzing the biomarker data, specifically the pupil dilation data, to determine the level of cognitive impairment of the user based on the correlation between pupil dilation and neuronal activity in the locus coeruleus. The method may also comprise providing feedback to the user or a clinician based on the level of cognitive impairment, such as a diagnosis, a prognosis, a recommendation, and/or a treatment plan.

The method can be implemented using various devices that can display cognitive tasks and measure pupil size, such as smartphones, tablets, computers, virtual reality headsets, augmented reality glasses, or wearable devices. The method can also use various techniques to analyze the pupil size data, such as machine learning, artificial intelligence, neural networks, statistical methods, or physiological models. The method can be used for various purposes, such as screening for cognitive impairment, monitoring cognitive decline, evaluating cognitive interventions, or enhancing cognitive performance. The method may also be used for prediction treatment response when a pharmacological and/or brain stimulation is applied.

The method is based on the principle that pupil size changes reflect brain activity in the locus coeruleus (LC), a brain region that regulates cognitive processes such as attention, memory, learning, and decision making. The LC is also affected by various cognitive disorders, such as depression, anxiety, Alzheimer's disease, Parkinson's disease, or schizophrenia. By measuring pupil size changes during or after cognitive tasks, the method can capture subtle changes in LC activity that may indicate cognitive impairment or improvement. As such the present invention can be used for various purposes, such as screening for cognitive impairment, monitoring cognitive decline, evaluating cognitive interventions, and/or enhancing cognitive performance.

Feedback provided based on the biomarker data can include a diagnosis of a specific neuropsychiatric and neurological disorder (e.g., depression, AD, etc.), a prognosis of the progression or severity of the cognitive impairment, a recommendation of a suitable cognitive intervention (e.g., cognitive training, medication, etc.), or a treatment plan that monitors the effectiveness of the intervention. The feedback can be provided in various forms, such as a score, a graph, a message, an alert, an audiovisual cue, a notification, or a report.

The method is advantageous over conventional methods of assessing cognitive function, such as neuropsychological tests or neuroimaging techniques, because it is simple, non-invasive, inexpensive, portable, and sensitive. The method can also provide objective and reliable measures of cognitive function. The task component can be configured to provide additional pupillometric tasks to determine subjective factors, in particular such as mood, sleepiness. The processing component can be configured to determine a contribution of the subjective factors on the biomarker response profile, respectively cognitive capability measure, which was determined based on the initial cognitive task.

The present invention can also use physiological models that account for factors such as age, gender, circadian rhythm, or medication that may affect pupil size.

The method can comprise the step of determining a profile parameter based on the biomarker response profile. The method can comprise the step of assessing the neurocognitive capability of the user based on the profile parameter. The method can comprise the step of mapping the profile parameter to a value on a cognitive capability scale to provide a cognitive capability measure. The method can comprise the step of determining a difference measure and assessing the neurocognitive capability of the user based on the difference measure. The method can comprise the step of step of determining a neurocognitive capability measure based on the biomarker response profile and/or the difference measure. The method can comprise the step of mapping the profile parameter to a value on a cognitive capability scale to provide a cognitive capability measure. The method can comprise the step of comparing the difference measure and/or the biomarker response profile against a set of predetermined values and associating the biomarker response profile with a neurocognitive capability class, which in particular corresponds to a pathological cognitive impairment of the user. The method can comprise the step of updating the cognitive capability measure while the cognitive task is provided, preferably while the cognitive task is performed by the user. The method can comprise the step of providing a confidence measure corresponding to a variability of the neurocognitive capability measure, in particular a variance and/or error measure. The method can comprise the step of providing a representation of the neurocognitive capability measure periodically or continuously, in particular parallel to the task component providing the cognitive task. The method can comprise the steps of storing a plurality of biomarker response profiles and/or cognitive capability measures and comparing a present biomarker response profile to the plurality of response profiles and to generate a relative measure of the biomarker response profile indicating a relation of the present biomarker response profile to the plurality of response profiles. The method can comprise the steps of receiving medical data relating to the user to which the task is provided and providing a relevance indicator corresponding to the cognitive capability measure in relation to the medical data.

The method can comprise the steps of providing a first task and a second task wherein the second task is configured to induce a higher cognitive load than the first task. The method can comprise the step of providing a plurality of tasks wherein each of the tasks of the plurality of tasks has an assigned cognitive load value and wherein the tasks are provided based on a predetermined cognitive load profile. The method can comprise the step of selecting a cognitive load profile from a plurality of cognitive load profiles. The method can comprise the step of generating a task based on a cognitive task template. The method can comprise the step of selecting a cognitive task template from a plurality of task templates. The method can comprise the step of selecting a task template and/or a task based on biomarker data and/or a biomarker response profile captured in response to a previously performed task. The method can comprise the step of selecting a task template and/or a task based on a predetermined expected quantitative difference in cognitive load of the respective task template and/or task, in particular in reference to a previously and/or subsequently provided task. The method can comprise the step of providing a working memory task, in particular an N-back task. The method can comprise the step of providing a set of tasks, preferably a set of consecutive N-back tasks, more preferably a 0-back, 1-back and 2-back task. The method can comprise the step of capturing an image of the eye of the user, preferably the iris of the user, and to determine the pupil size based on the captured image and wherein the pupil size is the biomarker. The method can comprise the step of determining the biomarker with a sampling rate greater or equal to a predetermined threshold rate of 1 Hz, 10 Hz, 25 Hz, 50 Hz or 100 Hz. The method can comprise the step of capturing the biomarker during a time interval associated with the cognitive task.

The method can comprise the step of determining the biomarker for a first time interval prior to the task being provided to the user, for a second time interval during which the task is provided to the user and/or for a third time interval after the cognitive task is no longer provided to the user. The method can comprise the step of determining a focus area based on the eye movement, and preferably determining if the focus area coincides with a display area providing the task or at least partially coincides with the display area. The method can comprise the step of at least partially blocking ambient light from entering the field of vision of the user. The method can comprise the step of determining a cognitive load of a provided cognitive task. The method can comprise the step of determining a baseline level of the biomarker associated with a baseline cognitive load level. The method can comprise the step of determining a maximum level of the biomarker associated with a maximum cognitive load level. The method can comprise the step of determining at least one intermediate level of the biomarker associated with a corresponding intermediate cognitive load level.

The method can comprise the step of interpolating and/or extrapolating a quantitative scale of the cognitive load, in particular wherein the cognitive load corresponds a representative biomarker value. The method can comprise the step of providing a set of tasks, wherein a cognitive load value of subsequent tasks differs by a predetermined value based on the quantitative scale of the cognitive load. The method can comprise the step of determining the profile parameter based on the quantitative difference in cognitive load in subsequent tasks.

Below, system embodiments will be discussed. These embodiments are abbreviated by the letter "S" followed by a number. Whenever reference is herein made to "system embodiments", these embodiments are meant.
S1. System (100) for assessing neurocognitive functioning, comprising
   a task component (101) configured to provide a task, in particular a cognitive task to a user;
   a capture component (102) configured to capture biomarker data of a biomarker of the user in response to the task;
   a processing component (103) configured to generate a biomarker response profile based on the biomarker data.
S2. System according to the preceding embodiment, wherein the processing component is configured to determine a profile parameter based on the biomarker response profile and to assess the neurocognitive capability of the user based on the profile parameter.
S3. System according to the preceding embodiment wherein, the profile parameter corresponds to a statistical measure and/or a parameter of a mathematical function, in particular a function fitted to the biomarker response profile.
S4. System according to any of the preceding embodiments comprising features of S2, wherein the processing component is configured to determine a difference measure and to assess the neurocognitive capability of the user based on the difference measure.
S5. System according to the preceding embodiment, wherein the difference measure corresponds to at least one of the following:
   difference of the profile parameter to a predetermined baseline value;
   difference of a fitted curve parameter to a predetermined fitted curve parameter;
   similarity of the biomarker response profile to a reference profile;
   scaling of the biomarker response profile in comparison to a reference profile;
   scaling of a parameter fitted to the biomarker response profile in comparison to a reference parameter.
S6. System according to any of the preceding embodiments comprising features of S3, wherein the processing component is configured to determine a neurocognitive capability measure based on the biomarker response profile and/or the difference measure.
S7. System according to any of the preceding embodiment, wherein the processing component is configured to compare the difference measure and/or the biomarker response profile against a set of predetermined values and to associate the biomarker response profile with a neurocognitive capability class, which in particular corresponds to a pathological cognitive impairment of the user.
S8. System according to any of the preceding embodiments, wherein the processing component is configured to map the profile parameter to a value on a cognitive capability scale to provide a cognitive capability measure.
S9. System according to any of the preceding embodiment, wherein the processing component is configured to update the cognitive capability measure while the cognitive task is provided, preferably while the cognitive task is performed by the user.
S10. System according to any of the preceding embodiments, wherein the processing component is configured to update the biomarker response profile periodically or continuously.
S11. System according to the preceding embodiment, wherein the processing component is configured to update the biomarker response profile while the cognitive task is provided, preferably while the cognitive task is performed by the user.
S12. System according to any of the preceding embodiments comprising features of S6, wherein the processing component is configured to update the neurocognitive capability measure periodically or continuously, in particular when a task is completed, sub-part of the task is completed or at least part of the task is completed.
S13. System according to the preceding embodiment, wherein the processing component is configured to provide the neurocognitive capability measure, in particular to a review component.
S14. System according to any of the preceding embodiments, wherein the processing component is configured to provide a confidence measure corresponding to a variability of the neurocognitive capability measure, in particular a variance and/or error measure.
S15. System according to any of the preceding embodiments, comprising the review component (106) configured to provide a representation of the biomarker response profile, in particular a numerical and/or graphical representation.
S16. System according to the preceding embodiment, wherein the review component is configured to provide a representation of the neurocognitive capability measure and/or the confidence measure.
S17. System according to the preceding embodiment, wherein the review component is configured to provide the representation of the neurocognitive capability measure periodically or continuously, in particular parallel to the task component providing the cognitive task.
S18. System according to any of the preceding embodiments comprising features of S15, wherein the review component is configured to provide task data to the task component and wherein the task component is configured to modify the current cognitive task based on the task data.
S19. System according to any of the preceding embodiments comprising features of S15, wherein the task component is configured to adjust a cognitive task difficulty of the cognitive task and/or provide a subsequent task with a cognitive task difficulty different to the cognitive task difficulty of the previous task.
S20. System according to any of the preceding embodiments, comprising a database module (105) configured to store a plurality of biomarker response profiles and/or cognitive capability measures.
S21. System according to the preceding embodiment, wherein the processing component is configured to compare a present biomarker response profile to the plurality of response profiles and to generate a relative measure of the biomarker response profile indicating a relation of the present biomarker response profile to the plurality of response profiles.
S22. System according to any of the preceding embodiments comprising features of S20, wherein the processing component is configured to compare a present cognitive capability measure to the plurality of cognitive capability measures and to generate a measure, in particular a relative measure, of the present cognitive capability measure indicating a relation of the present cognitive capability measure to the plurality of cognitive capability measures.
S23. System according to any of the preceding embodiments, wherein the processing component is configured to receive medical data relating to the user to which the task is provided and to provide a relevance indicator corresponding to the cognitive capability measure in relation to the medical data.
S24. System according to any of the preceding embodiments, wherein the task component is configured to provide a first task and a second task and wherein the second task is configured to induce a higher cognitive load than the first task.
S25. System according to any of the preceding embodiments, wherein the task component is configured to provide a task comprising a plurality of task conditions, preferably, wherein the task component is configured to provide at least two subsequent task conditions, in particular wherein the second task condition is configured to induce a higher cognitive load than the first task.
S26. System according to any of the preceding embodiments, wherein the task component is configured to provide a plurality of tasks wherein each of the tasks of the plurality of tasks has an assigned cognitive load value and wherein the tasks are provided based on a predetermined cognitive load profile.
S27. System according to any of the preceding embodiments, wherein the task component is configured to select a cognitive load profile from a plurality of cognitive load profiles.
S28. System according to any of the preceding embodiments, wherein the task component is configured to generate a task based on a cognitive task template, preferably wherein the task template comprises a predetermined sequence of task conditions.
S29. System according to any of the preceding embodiments, wherein the task component is configured to select a cognitive task template from a plurality of task templates.
S30. System according to the preceding embodiment, wherein the task component is configured to select a task template and/or a task based on biomarker data and/or a biomarker response profile captured in response to a previously performed task.
S31. System according to the preceding embodiment, wherein the task component is configured to select a task template and/or a task based on a predetermined expected quantitative difference in cognitive load of the respective task template and/or task, in particular in reference to a previously and/or subsequently provided task.
S32. System according to any of the preceding embodiments, wherein the task component is configured to provide a working memory task, in particular an N-back task.
S33. System according to any of the preceding embodiments, wherein the task component is configured to provide a set of tasks, preferably a set of working memory tasks, more preferably a set of consecutive N-back tasks, i.e., a 0-back, 1-back, 2-back and/or 3-back task.
S34. System according to the preceding embodiment, wherein the task component is configured to provide a fixation task and/or a digit span task, in particular as part of the plurality of tasks.
S35. System according to any of the preceding embodiments, wherein the biomarker corresponds to a pupil size of the user, and wherein the capture component comprises a camera module (104) configured to capture an image of the eye of the user, preferably the iris of the user, and to determine the pupil size based on the captured image.
S36. System according to any of the preceding embodiments, wherein the capture component comprises an illuminator configured to illuminate part of the user, preferably configured to illuminate the face and/or the eye of the user, more preferably configured to illuminate the iris and/or pupil of the user.
S37. System according to the preceding embodiment, wherein the illuminator is configured to illuminate both eyes, irises and/or pupils of the user.
S38. System according to any of the preceding embodiments comprising features of S36, wherein the illuminator is configured to emit light in the visible spectrum and/or the infrared spectrum, preferably within the range of 700 nm to 1000 nm, preferably 850 nm to 950 nm, more preferably 890 nm or 940 nm.
S39. System according to any of the preceding embodiments, wherein the capture component is configured to continuously determine the biomarker with a sampling rate greater or equal to a predetermined threshold rate of 1 Hz, 10 Hz, 25 Hz, 50 Hz or 100 Hz.
S40. System according to any of the preceding embodiments, wherein the capture component is configured to determine a relative value change of the biomarker.
S41. System according to any of the preceding embodiments, wherein the capture component comprises a sensor configured to capture the biomarker data, in particular wherein the processing component is configured to determine the biomarker with a relative accuracy of 5%, preferably 1%, more preferably 0.1% based on the biomarker data.
S42. System according to any of the preceding embodiments, wherein the capture component is configured to capture the biomarker during a time interval associated with the cognitive task.
S43. System according to any of the preceding embodiments, wherein the capture module is configured to determine the biomarker for a first time interval prior to the task being provided to the user, for a second time interval during which the task is provided to the user and/or for a third time interval after the cognitive task is no longer provided to the user.
S44. System according to any of the preceding embodiments comprising features of S41, wherein the sensor is configured to track a pupil size of the user.
S45. System according to any of the preceding embodiments comprising features of S41, wherein the sensor is configured to track an eye movement of the user.
S46. System according to any of the preceding embodiments, wherein the processing component is configured to determine a focus area based on the eye movement, preferably wherein the processing component is configured to determine if the focus area coincides with a display area providing the task or at least partially coincides with the display area.
S47. System according to any of the preceding embodiments, comprising a display component (107) configured to display the task to the user.
S48. System according to the preceding embodiment, comprising a headset component (108) configured to mount onto the head of a user and to dispose the display component at least partially in the field of vision of the user, preferably to dispose a primary area defined by the task within the field of vision of the user.
S49. System according to the preceding embodiment, wherein the headset component is configured to at least partially block ambient light from entering the field of vision of the user.
S50. System according to any of the preceding embodiments, wherein the display component comprises two display units configured to display the task in conjunction with one another, where each of the display units is associated with one eye of the user.
S51. System according to any of the preceding embodiments, comprising a calibration component (109) configured to determine a cognitive load of a provided cognitive task.
S52. System according to the preceding embodiment, wherein the calibration component is configured to determine a baseline level of the biomarker associated with a baseline cognitive load level.
S53. System according to any of the preceding embodiments comprising features of S51, wherein the calibration component is configured to determine a maximum level of the biomarker associated with a maximum cognitive load level.
S54. System according to any of the preceding embodiments comprising features of S51, wherein the calibration component is configured to determine at least one intermediate level of the biomarker associated with a corresponding intermediate cognitive load level.
S55. System according to any of the preceding embodiments comprising features of S51, wherein the calibration component is configured to generate, in particular to interpolate and/or to extrapolate, a scale of the cognitive load, in particular wherein the cognitive load corresponds a representative biomarker value.
S56. System according to the preceding embodiment, wherein the task component is configured to provide a set of tasks, wherein a cognitive load, preferably a cognitive load value, of subsequent tasks is predetermined to increase or to decrease, preferably by a predetermined value, based on the scale of the cognitive load.
S57. System according to the preceding embodiment comprising features of S8, wherein the processing component is configured to determine the profile parameter based on the quantitative difference in cognitive load in subsequent tasks.

Below, method embodiments will be discussed. These embodiments are abbreviated by the letter "M" followed by a number. Whenever reference is herein made to "method embodiments", these embodiments are meant.
M1. Method for assessing neurocognitive functioning, comprising the steps of
   providing a task, in particular a cognitive task to a user;
   capturing biomarker data of a biomarker of the user;
   generating a biomarker response profile based on the biomarker data.
M2. Method according to the preceding embodiment comprising the step of determining a profile parameter based on the biomarker response profile.
M3. Method according to any of the preceding embodiments comprising the step of assessing the neurocognitive capability of the user based on the profile parameter.
M4. Method according to any of the preceding embodiments comprising the step of mapping the profile parameter to a value on a cognitive capability scale to provide a cognitive capability measure.
M5. Method according to any of the preceding embodiments comprising the step of determining a difference measure and assessing the neurocognitive capability of the user based on the difference measure.
M6. Method according to the preceding embodiment comprising the step of determining a neurocognitive capability measure based on the biomarker response profile and/or the difference measure.
M7. Method according to any of the preceding embodiments comprising the step of mapping the profile parameter to a value on a cognitive capability scale to provide a cognitive capability measure.
M8. Method according to any of the preceding embodiments comprising features of M6, further comprising the step of comparing the difference measure and/or the biomarker response profile against a set of predetermined values and associating the biomarker response profile with a neurocognitive capability class, which in particular corresponds to a pathological cognitive impairment of the user.
M9. Method according to any of the preceding embodiments comprising the step of updating the cognitive capability measure while the cognitive task is provided, preferably while the cognitive task is performed by the user.
M10. Method according to any of the preceding embodiments comprising the step of providing a confidence measure corresponding to a variability of the neurocognitive capability measure, in particular a variance and/or error measure.
M11. Method according to any of the preceding embodiments comprising the step of providing a representation of the neurocognitive capability measure periodically or continuously, in particular parallel to the task component providing the cognitive task.
M12. Method according to any of the preceding embodiments comprising the steps of storing a plurality of biomarker response profiles and/or cognitive capability measures and comparing a present biomarker response profile to the plurality of response profiles and to generate a relative measure of the biomarker response profile indicating a relation of the present biomarker response profile to the plurality of response profiles.
M13. Method according to any of the preceding embodiments comprising the steps of receiving medical data relating to the user to which the task is provided and providing a relevance indicator corresponding to the cognitive capability measure in relation to the medical data.
M14. Method according to any of the preceding embodiments comprising the steps of providing a first task and a second task wherein the second task is configured to induce a higher cognitive load than the first task.
M15. Method according to any of the preceding embodiments comprising the step of providing a plurality of tasks wherein each of the tasks of the plurality of tasks has an assigned cognitive load value and wherein the tasks are provided based on a predetermined cognitive load profile.
M16. Method according to any of the preceding embodiments comprising the step of selecting a cognitive load profile from a plurality of cognitive load profiles.
M17. Method according to any of the preceding embodiments comprising the step of generating a task based on a cognitive task template.
M18. Method according to any of the preceding embodiments comprising the step of selecting a cognitive task template from a plurality of task templates.
M 19. Method according to any of the preceding embodiments comprising the step of selecting a task template and/or a task based on biomarker data and/or a biomarker response profile captured in response to a previously performed task.
M20. Method according to any of the preceding embodiments comprising the step of selecting a task template and/or a task based on a predetermined expected quantitative difference in cognitive load of the respective task template and/or task, in particular in reference to a previously and/or subsequently provided task.
M21. Method according to any of the preceding embodiments comprising the step of providing a working memory task, in particular an N-back task.
M22. Method according to any of the preceding embodiments comprising the step of providing a set of tasks, preferably a set of consecutive N-back tasks, more preferably a 0-back, 1-back and 2-back task.
M23. Method according to any of the preceding embodiments comprising the step of capturing an image of the eye of the user, preferably the iris of the user, and to determine the pupil size based on the captured image and wherein the pupil size is the biomarker.
M24. Method according to any of the preceding embodiments comprising the step of determining the biomarker with a sampling rate greater or equal to a predetermined threshold rate of 1 Hz, 10 Hz, 25 Hz, 50 Hz or 100 Hz.
M25. Method according to any of the preceding embodiments comprising the step of capturing the biomarker during a time interval associated with the cognitive task.
M26. Method according to any of the preceding embodiments comprising the step of determining the biomarker for a first time interval prior to the task being provided to the user, for a second time interval during which the task is provided to the user and/or for a third time interval after the cognitive task is no longer provided to the user.
M27. Method according to any of the preceding embodiments comprising the step of determining a focus area based on the eye movement, and preferably determining if the focus area coincides with a display area providing the task or at least partially coincides with the display area.
M28. Method according to any of the preceding embodiments comprising the step of at least partially blocking ambient light from entering the field of vision of the user.
M29. Method according to any of the preceding embodiments comprising the step of determining a cognitive load of a provided cognitive task.
M30. Method according to any of the preceding embodiments comprising the step of determining a baseline level of the biomarker associated with a baseline cognitive load level.
M31. Method according to any of the preceding embodiments comprising the step of determining a maximum level of the biomarker associated with a maximum cognitive load level.
M32. Method according to any of the preceding embodiments comprising the step of determining at least one intermediate level of the biomarker associated with a corresponding intermediate cognitive load level.
M33. Method according to any of the preceding embodiments comprising the step of interpolating and/or extrapolating a quantitative scale of the cognitive load, in particular wherein the cognitive load corresponds a representative biomarker value.
M34. Method according to any of the preceding embodiments comprising the step of providing a set of tasks, wherein a cognitive load value of subsequent tasks differs by a predetermined value based on the quantitative scale of the cognitive load.
M35. Method according to any of the preceding embodiments comprising the step of determining the profile parameter based on the quantitative difference in cognitive load in subsequent tasks.

Below, embodiments of a computer program product will be discussed. These embodiments are abbreviated by the letter "C" followed by a number. Whenever reference is herein made to the "computer program product embodiments", these embodiments are meant.
C1. A computer program product comprising instructions, which, when executed by a system and any of its components according to any of the system embodiments, cause the system and its components to perform the steps, for which the system and/or any of its components are configured.
C2. A computer program product comprising instructions, which, when executed by the system and any of its components according to any of the system embodiments, cause the system and its respective components to perform the steps of the method according to any of the method embodiments, which method steps are performed by the system and/or the respective components according to the method. Below, use embodiments will be discussed. These embodiments are abbreviated by the letter "U" followed by a number. Whenever reference is herein made to "use embodiments", these embodiments are meant.
U1. Use of the system according to any of the preceding system embodiments, for carrying out the method according to any of the preceding method embodiments.
U2. Use of the method according to any of the preceding method embodiments and the system according to any of the preceding embodiments, for detection of neurocognitive functioning, in particular in users with cognitive impairments, specifically cognitive impairments due to psychopathological syndromes and/or neurodegenerative syndromes.

### Brief description of figures

The present invention will now be described with reference to the accompanying drawings, which illustrate embodiments of the invention. These embodiments should only exemplify, but not limit, the present invention.
- Fig. 1: schematically depicts a drawing of an embodiment of a system according to the invention;
- Fig. 2: schematically depicts a correlation of a biomarker and cognitive load according to the invention;
- Fig. 3: schematically depicts a drawing of a temporal sequence of cues provided to the user as part of a cognitive tasks according to the invention;
- Fig. 4: schematically depicts a drawing of biomarker response profiles according to the invention; and
- Fig. 5: schematically depicts a drawing of biomarker values corresponding to cognitive loads according to the invention.

### Detailed description of Figures

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

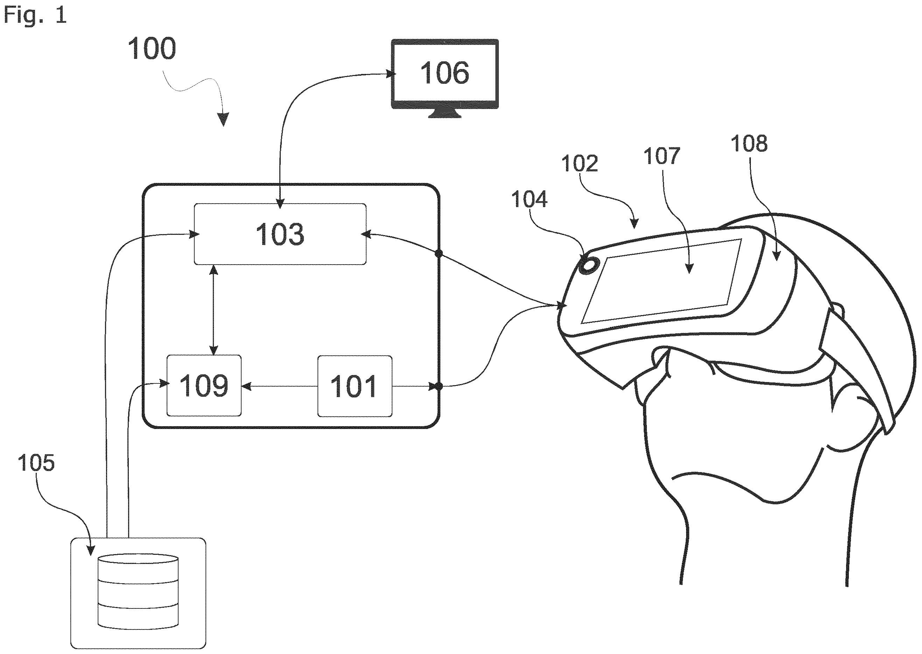

**Figure 1** is a schematic drawing of an embodiment of the system 100 according to the invention. The system can comprise a task component 101, a capture component 102, and a processing component 103. The task component can be configured to provide a cognitive task to the user, such as a memory, attention, or reasoning task. The capture component can be configured to capture biomarker data of a biomarker of the user, i.e., a pupil dilation. The biomarker can also correspond to brain activity, eye movement, heart rate, actigraphy, and/or skin conductance. Actigraphy can be performed by the VR headset component. The processing component can be configured to generate a biomarker response profile based on the biomarker data. The biomarker response profile reflects how the user's biomarker changes in response to the cognitive task.

The processing component is further configured to assess the neurocognitive capability of the user based on the biomarker response profile. To do so, the processing component can be configured to perform one or more of the following functions:
- Determining a profile parameter based on the biomarker response profile and compare it to a predetermined baseline value. The profile parameter can be a statistical measure (such as mean, standard deviation, or correlation) or a parameter of a mathematical function (such as slope, intercept, or curvature) fitted to the biomarker response profile. The difference between the profile parameter and the baseline value can indicate the level of neurocognitive capability of the user. Preferably, the profile parameter can relate to one of the following quantities: variance, standard deviation, first derivative of pupil dilation, first derivative of pupil size.
- Determining a difference measure based on the similarity or scaling of the biomarker response profile or a parameter fitted to it in comparison to a reference profile or a reference parameter. The reference profile or parameter can represent a normative or expected biomarker response for a given cognitive task. The difference measure can indicate how much the user's biomarker response deviates from the reference.
- Mapping the profile parameter or the difference measure to a value on a cognitive capability scale to provide a cognitive capability measure. The cognitive capability scale can be a numerical or categorical scale that reflects different levels of neurocognitive functioning, such as normal, mild impairment, moderate impairment, or severe impairment.
- Comparing the difference measure and/or the biomarker response profile against a set of predetermined values and associate the biomarker response profile with a neurocognitive capability class. The neurocognitive capability class can correspond to a pathological cognitive impairment of the user, such as occurring within Alzheimer's disease, Parkinson's disease, or neuropsychiatric disorders.

The processing component can update the biomarker response profile, the difference measure, and/or the cognitive capability measure periodically or continuously while the cognitive task is provided or performed by the user. This can allow for real-time monitoring and assessment of neurocognitive functioning.

The system can also comprise a review component 106 configured to provide a representation of the biomarker response profile, the difference measure, and/or the cognitive capability measure. The representation can be numerical and/or graphical and can be provided periodically or continuously, in particular parallel to the task component providing the cognitive task. The review component can also provide a confidence measure corresponding to a variability of the cognitive capability measure, such as a variance or an error measure. The representation and the confidence measure can help evaluate the reliability and validity of the neurocognitive assessment.

The system can also be configured to provide feedback and adaptation of the cognitive task based on the biomarker response profile and/or the cognitive capability measure. Thereto, the system can be configured to execute one of the following functions:
- Providing task data to the task component from the review component and modifying the current cognitive task based on the task data. The task data can include information about the user's performance, engagement, or preference for the cognitive task.
- Adjusting the cognitive task difficulty of the current or subsequent cognitive task based on the biomarker response profile and/or the cognitive capability measure. The cognitive task difficulty can be increased or decreased to match the user's neurocognitive level and to provide an optimal challenge for the user.

A plurality of biomarker response profiles and/or cognitive capability measures can be stored in a database module (105). The presently captured biomarker response profile or cognitive capability measure can be compared to stored biomarker response profiles, respectively cognitive capability measures. The comparison can generate a relative measure of the present biomarker response profile or cognitive capability measure indicating how it relates to the stored ones. The relative measure can help identify trends, patterns, or outliers in the user's neurocognitive functioning over time or across different tasks.

The system can be configured to receive medical data relating to the user, such as diagnosis, medication, symptoms, and/or other biomarkers, and provide a relevance indicator corresponding to the cognitive capability measure in relation to the medical data. The relevance indicator can help evaluate how the user's neurocognitive functioning is affected by or affects their medical condition.

The system can also be configured to provide different types of cognitive tasks with different levels of cognitive load. Cognitive load refers to the amount of mental effort required to perform a cognitive task. A first task and a second task can be provided, where the second task is configured to induce a higher cognitive load than the first task.

The task can be a working memory task, such as an N-back task, where the user has to remember a sequence of stimuli presented on a screen and indicate whether the current stimulus matches the one presented N steps back. The level of cognitive load can vary depending on the value of N (e.g., 0-back, 1-back, 2-back, or 3-back as well as a fixation condition).

Tasks can also be provided according to a predetermined cognitive load profile. The cognitive load profile can specify how the level of cognitive load changes over time or across tasks. For example, the cognitive load profile can be increasing, decreasing, constant, variable, pseudo-randomly or randomly interspersed. The system can select a cognitive load profile from a plurality of predefined profiles or generate a custom profile based on user input or feedback.

Tasks can be generated based on a cognitive task template that can define the structure, content, and parameters of a cognitive task. For example, the cognitive task template can specify what type of stimuli (e.g., words, numbers, images, sounds) are used, how they are presented (e.g., duration, frequency, order), and what type of response (e.g., verbal, manual, eye movement) is required from the user. The system can select a cognitive task template from a plurality of predefined templates or generate a custom template based on user input or feedback.

The system can be configured to tailor the cognitive task to the user's neurocognitive state and preferences, in particular based on previously captured biomarker data, respectively a previously captured biomarker response profile.

The system can control how much the level of cognitive load changes between tasks. For example, if the system may be configured to increase or decrease the level of cognitive load gradually or abruptly.

The system can also be configured to capture different types of biomarkers from different sources. Biomarkers are biological indicators that reflect physiological processes related to neurocognitive functioning.

The system can comprise an illuminator to illuminate part of the user, preferably the face and/or the eye of the user, more preferably the iris and/or pupil of the user. The illuminator can be integrated into the headset component, specifically the illuminator can be part of a VR-headset. The illuminator can help improve the quality and accuracy of the image captured by the camera module. Preferably, the illuminator can be configured to illuminate both eyes, irises, and/or pupils of the user. The illuminator can be configured to emit infrared light that can help reduce the interference from ambient light and increase the contrast between the iris and the pupil.

The processing component can be configured to normalize biomarker data across different users or sessions and account for individual differences or variations in baseline levels or measurement conditions.

The system can also be configured to provide an immersive and interactive experience for the user by using a virtual reality (VR) headset. The VR headset can create a simulated environment that surrounds and responds to the user's actions. The system can comprise a display component (107) to display the cognitive task to the user. The display component can present visual stimuli that are relevant to the cognitive task, such as words, numbers, images, or shapes. The display component can also provide feedback or instructions to guide or motivate the user during or after the cognitive task.

A headset component (108) can mount onto the head of the user and dispose the display component at least partially in the field of vision of the user, preferably to dispose a primary area defined by the cognitive task within the field of vision of the user. The headset component can help position and stabilize the display component in relation to the user's eyes and head movements. The headset component can also help isolate or block ambient light from entering the field of vision of the user and reduce distractions or interference from the external environment.

The display component can be comprised of two display units that can create a stereoscopic effect that enhances the depth perception and/or increases realism of the visual stimuli. For example, the displayed task may be perceived as a three-dimensional representation. The VR headset can immerse the user in a realistic and interactive environment. The system can also use the feedback component to provide the patient with adaptive and personalized training tasks that target their specific cognitive impairments and improve their functional outcomes.

The system may also comprise a heart rate monitor, a skin conductance sensor and/or an actigraphy, i.e., an actimetry sensor, to capture the user's heart rate, skin conductance and/or motor activity as biomarkers of emotional arousal and anxiety. The system can apply a correction and/or weight to the biomarker response profile according to the additional biomarkers.

The system can comprise a smart device which is used as an output interface for the task component, respectively the processing component. The smart device may comprise sensors, in particular imaging devices, configured to capture images of the pupil. A local processor of the smart device can be configured to preprocess the captured data. The preprocessed data may be provided to the processing component for analysis and generating the biomarker response profile. Herein, preprocessing may increase the data quality, i.e. an SNR increase, image processing and/or may increase data transfer efficiency in compressing the captured data prior to transfer of the data to the processing component. Alternatively, the local processor of the smart device can be the processing component such that evaluating the biomarker data can be achieved locally. The preprocessing and/or processing of the data may comprise interpolation to account for missing or low-quality data, e.g., caused by blinks, and/or further comprise smoothing, filtering of the data and/or standardization, e.g., z-scoring.

The system may comprise a decentralized communication infrastructure. Specifically, the processing component, task component and/or database module can be cloud based or remotely located components.

The processing component can be configured to determine an inconsistency in the biomarker data, i.e., blinking when capturing pupil dilation. The processing component can be configured to adjust the task based on detecting an inconsistency. For example, the processing component can provide task data to the task component, wherein the task data indicates a repeat section of the corresponding task section that was affected by the inconsistency.

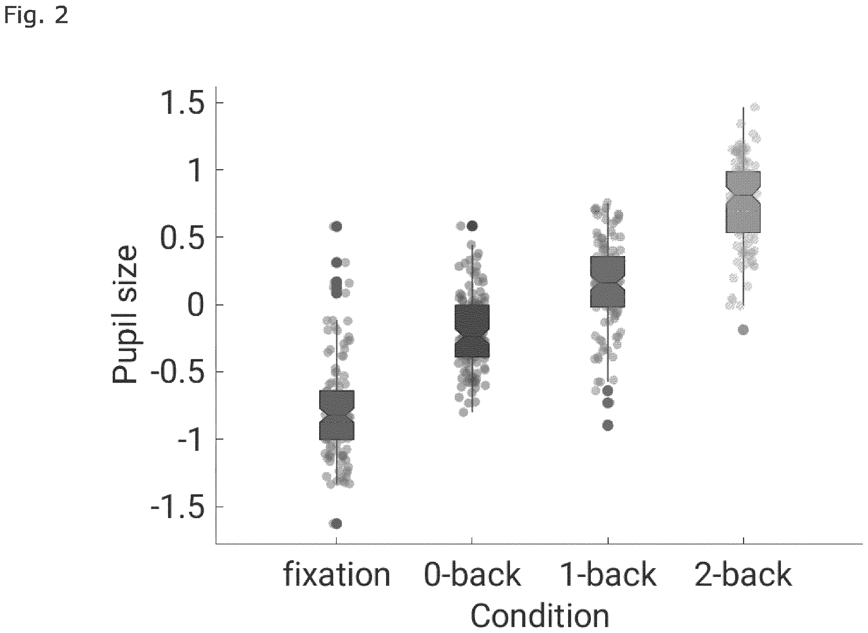

**Figure 2** schematically depicts a Boxplot showing the distribution of pupil size calculated as the mean of pupil size values in each condition (one value per user). During an N-back task user's may be provided with a sequence of stimuli (e.g., letters), preferably appearing one after another and are tasked to respond whenever a current stimulus, i.e., target, matches the one from n steps earlier in the sequence. For example, a set of capital letters can be used as stimuli and a task may comprise a plurality of blocks, each consisting of a plurality of stimuli, of the type 0-back, 1-back, 2-back, and/or fixation. Conditions with higher load can be applied.

Each task condition can be presented once in the first half of the task and once in the second half of the task. For example, a first half of the task can correspond to increasing cognitive load (0-back, 1-back, 2-back) followed by a fixation condition and a second half of the task can correspond to a decreasing cognitive load (2-back, 1-back, 0-back). Alternatively, an interspersed order of tasks can be implemented. During the 0-back condition, the user can be instructed to react with a button press when a single prespecified target letter (i.e., W) is displayed. The 0-back condition can be a control condition may cause attentional demand but may not create working memory demand (i.e., minimal working memory load). In the 1-back and 2-back conditions, users can be instructed to indicate, preferably via a button press whether a letter presented on the screen (= target) matches a letter one step before, respectively two steps before. The cognitive load can increase from the 1-back condition to the 2-back condition.

In a fixation condition, a predetermined capital letter can be provided to the user, i.e., displayed repeatedly as a substitution for the letter stimuli on the screen. The fixation condition may carry the lowest cognitive load. Furthermore, fixation tasks can be provided which comprise presenting a fixation point (such as a cross or a dot) to the user and asking them to maintain their gaze on it, preferably while ignoring distractors, e.g., flashing lights or sounds. Frequency, intensity, and/or location of the distractors, and/or the duration of the task can affect the biomarker response profile. The fixation condition may be provided without distractors.

The user may be provided with the task instruction that no response is required, specifically no conditional response. This condition can serve as a baseline control as it includes a visual input but does not prompt a motor response as well as a working memory and/or recognition, respectively attentional aspect.

The horizontal line within each box denotes the respective median value. The boxes extend from the 25th to 75th percentile. The vertical extending lines (whiskers) denote values outside the interquartile range (IQR). The upper whisker extends to the largest value no further than 1.5 IQR and the lower whisker extends to the smallest value no further than 1.5 IQR. Dots beyond the end of the whiskers represent outliers.

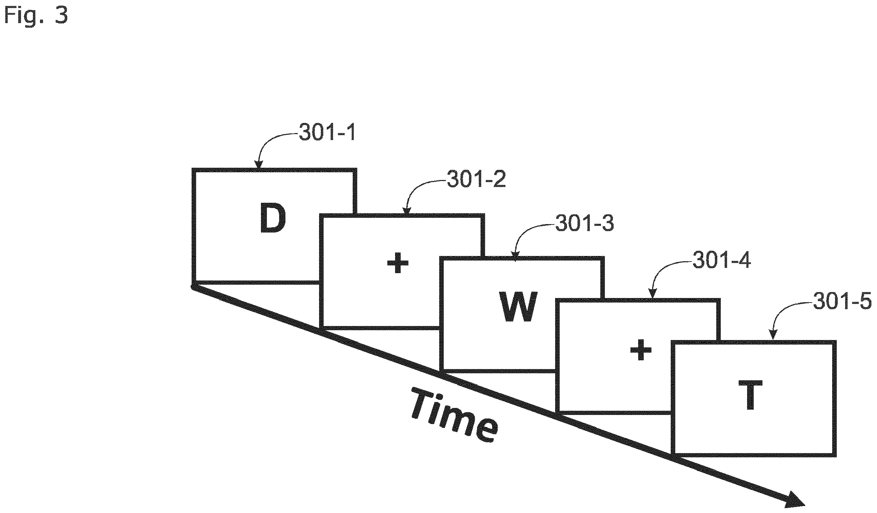

**Figure 3** is a schematic representation of a task provided to the user. The task may comprise visual cues 301-1 to 301-5, wherein each cue is provided to the user for a predetermined time interval. The cues can comprise a visual imprint, i.e. a letter, pattern, position, color etc., correspond to a specific task, i.e. an N-back task.

The task may require both storing and manipulating information in a short period of time. N-back tasks may comprise presenting a series of stimuli (such as letters, numbers, or images) to the user and asking them to indicate whether the current stimulus matches the one presented N steps back. The level of cognitive load can be varied by changing the value of N, the speed of presentation, or the type of stimuli.

Alternatively, spatial working memory tasks can be provided which comprise presenting a series of spatial locations, i.e., dots on a screen to the user wherein a response request comprises reproducing the spatial location, order, pattern and/or color. The level of cognitive load can be varied by varying the number, size, or arrangement of elements, i.e., dots, and/or the length or complexity of the sequence. ³

A cognitive load theory can be defined as changes in pupil size during cognitive tasks. More difficult tasks may require more mental resources. The pupil size can reflect the cognitive load in all individuals, specifically persons within the neurocognitive norm and neurocognitively impaired persons, in particular persons affected by Alzheimer's disease.

Pupil size variations can correspond to the involvement of the sympathetic and parasympathetic nervous systems, which regulate the arousal and relaxation of the body. The sympathetic system activates the muscles that dilate the pupil, while the parasympathetic system activates the muscles that constrict the pupil. Therefore, the larger pupil size during more difficult tasks, such as backward span can correlate to a higher sympathetic activity and lower parasympathetic activity. Equally, smaller pupil size during easier tasks, such as counting can correspond to lower sympathetic activity and higher parasympathetic activity. The parasympathetic system may also be associated with acetylcholine, a neurotransmitter that is impaired in Alzheimer's disease. Therefore, the pupil size variations can reflect both sympathetic and parasympathetic activities, which are both affected by Alzheimer's disease.

Thus, pupillometry, i.e., the measurement of pupil size, can be a used as a biomarker of cognitive processing in Alzheimer's disease. The present system does not rely on measuring the light response of the pupil but instead captures the cognitive response.

Users affected by Alzheimer's disease may show a reduced modulation in their pupil size across different tasks when compared to healthy comparison subjects. The pupil response may be overall reduced when exposed to different cognitive loads. A reduced modulation can be an indicator for increased cognitive load during tasks or task conditions associated with comparatively easier conditions, i.e., fixation or 0-back. These tasks may require a relatively larger pupil response, i.e. more effort compared to healthy controls. In contrast, more difficult conditions may produce a comparatively lower response. The combination of these effects can manifest as a levelling of the biomarker response profile.

Pupillometry during a cognitive task can accurately and sensitively cover cognitive effort, performance, and/or cognitive impairment of mildly to moderately depressed patients. The system may differentiate two groups of patients based on the captured biomarker data: a first group with a continuous increase in pupillary volume with increasing cognitive effort, and a second group that does not show this increase and at the same time has slightly reduced scores on several diagnostic neurocognitive tasks. The tasks may include the domains of attention, working memory, cognitive flexibility, and inhibition. Cognitive impairment may not be indicated by the task results alone. Pupillary response measurements during cognitive tasks can provide a sensitive and scalable biomarker of cognitive impairment.

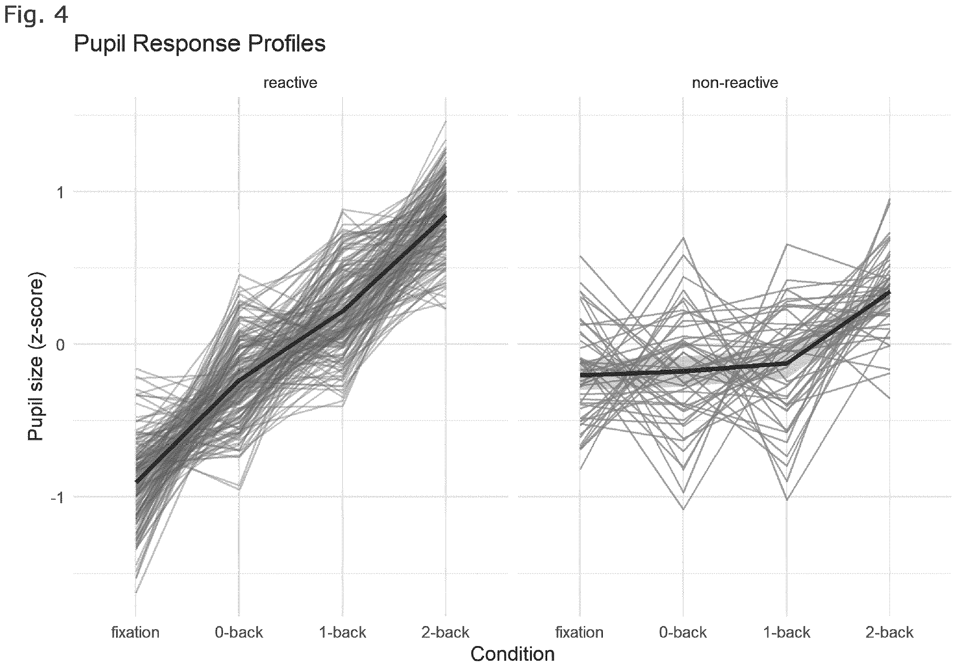

**Figure 4** shows biomarker response profiles for the first group (left) and for the second group (right) including a fit generated by the processing module. The second group can be characterized by as well-functioning psychiatric patients. Herein, Alzheimer's disease patients show less dilated pupils to cognitively challenging conditions such as the forward and backward spans compared to the first group.

The processing component can be configured to execute a LCGM clustering approach to test whether behavioural neurocognitive readouts lead to the same cluster solution as pupillometry. The processing component can be configured to determine a mean reaction time per condition of the N-back task

to identify the optimal number of classes, the processing component can be configured to fit models with increasing number of latent classes to the biomarker data, respectively the biomarker response profile. Thereto, the processing component can execute a predetermined number of iterations of the clustering model.

For the identification of the best fitting model the Bayesian Information Criterion (BIC) can be applied. The processing component can be configured to determine a stability measure of the cluster solution. Thereto the holdout method can be applied for validation purposes. The analysis can be repeated with randomly selected subsamples, e.g., leaving out a predetermined percentage of the participants.

The processing component can be configured to determine an individual mean accuracy rates of the N-back and providing these rates to the LCGM following the same procedure as for reaction time and mean pupil size.

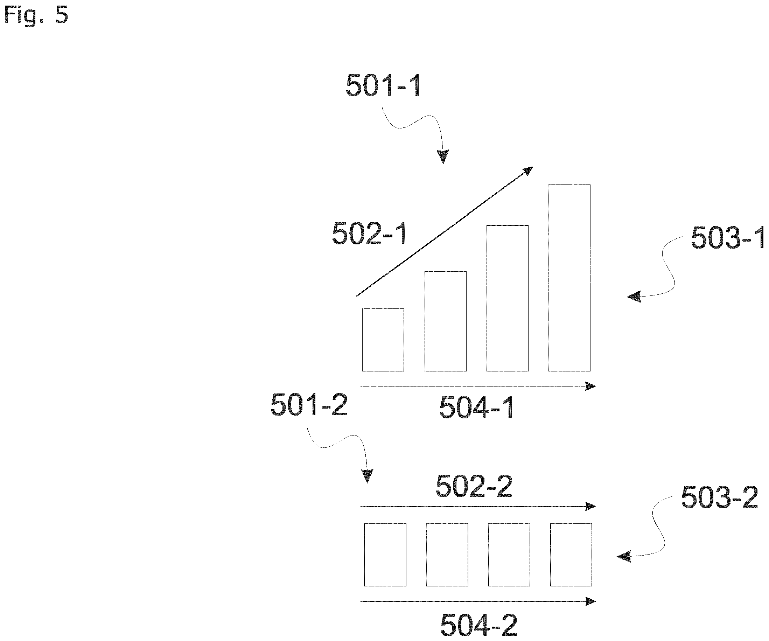

**Figure 5** schematically depicts a drawing of biomarker values corresponding to cognitive loads according to the invention. A first category profile 501-1 can be associated with a healthy condition of a user, respectively a group of users or a plurality of biomarker profiles determined for a single healthy individual. A second category profile 501-2 can be associated with a pathological condition of a user, respectively a group of users or a plurality of biomarker profiles determined for a single healthy individual. Herein, the two category profiles can be distinguished by a specific difference measure, respectively profile parameter. In this example, a slope or gradient of the function biomarker(cognitive load) can be determined and based on the respective value of the profile parameter, the corresponding biomarker response profile can be associated with a specific condition. The indicators 504-1. 504-2 represent an increasing cognitive load. The cognitive load and biomarker be processed as a functional mapping, where the biomarker is a function of the cognitive load. Thereto, the indicators 504-1, 504-2 can represent an X-axis and the cognitive load can be attributed to a corresponding Y-axis. The first biomarker indicator 502-1 corresponds to an increasing biomarker, i.e., pupil size, and the second biomarker indicator 502-2 corresponds to a stagnant biomarker. Each set of bars 503-1, 503-2 can represent a set of cognitive tasks or cognitive task conditions. Each set 503-1, 503-2 can be represent a sequence of increasing cognitive load, in particular without a quantified difference in cognitive load between the set elements. Alternatively, a quantitative difference between the set elements can be determined such that each element is positioned at specific value on a cognitive load scale.

## Claims

1. System (100) for assessing neurocognitive functioning, comprising
a task component (101) configured to provide a task to a user;
a capture component (102) configured to capture biomarker data of a biomarker of the user in response to the task;
a processing component (103) configured to generate a biomarker response profile based on the biomarker data.

2. System according to the preceding claim wherein the processing component is configured to determine a profile parameter based on the biomarker response profile and to assess the neurocognitive capability of the user based on the profile parameter.

3. System according to claim 2 wherein the processing component is configured to map the profile parameter to a value on a cognitive capability scale to provide a cognitive capability measure.

4. System according to any of the preceding claims comprising a review component (106) wherein the review component is configured to provide a representation of the neurocognitive capability measure.

5. System according to any of the preceding claims wherein the task component is configured to adjust a cognitive task difficulty of the cognitive task and to provide a subsequent task with a cognitive task difficulty different to the cognitive task difficulty of the previous task.

6. System according to any of the preceding claims comprising a database module (105) configured to store a plurality of biomarker response profiles and
wherein the processing component is configured to compare a present biomarker response profile to the plurality of response profiles and to generate a relative measure of the biomarker response profile indicating a relation of the present biomarker response profile to the plurality of response profiles.

7. System according to any of the preceding claims wherein the processing component is configured to receive medical data relating to the user to which the task is provided and to provide a relevance indicator corresponding to the cognitive capability measure in relation to the medical data.

8. System according to any of the preceding claims wherein the task component is configured to provide a task comprising a plurality of task conditions,
[S28] wherein the task component is configured to generate a task based on a cognitive task template, and wherein the task template comprises a predetermined sequence of task conditions.

9. System according to any of the preceding claims wherein the capture component comprises a sensor configured to capture the biomarker data, and
[S44] wherein the sensor is configured to track a pupil size as the biomarker of the user.

10. System according to any of the preceding claims comprising a display component (107) configured to display the task to the user and
comprising a headset component (108) configured to mount onto the head of a user and to dispose the display component at least partially in the field of vision of the user.

11. System according to any of the preceding claims comprising a calibration component (109) configured to determine a cognitive load of a provided cognitive task and wherein the calibration component is configured to determine a baseline level of the biomarker associated with a baseline cognitive load level.

12. System according to claim 11, wherein the calibration component is configured to generate a scale of the cognitive load, wherein the cognitive load corresponds to a representative biomarker value and
wherein the task component is configured to provide a set of tasks, wherein a cognitive load of subsequent tasks is predetermined to increase or to decrease or to vary in an interspersed manner based on the scale of the cognitive load.

13. Method for assessing neurocognitive functioning, comprising the steps of
providing a task, in particular a cognitive task to a user;
capturing biomarker data of a biomarker of the user;
generating a biomarker response profile based on the biomarker data.

14. Method according to claim 13 comprising the steps of
determining a profile parameter based on the biomarker response profile; and assessing the neurocognitive capability of the user based on the profile parameter.

15. A computer program product comprising instructions, which, when executed by the system and any of its components according to any of the claims 1 to 12, cause the system and its respective components to perform the steps of the method according to any of the claims 13 or 14, which method steps are performed by the system and/or the respective components according to the method.
